# NEW EUROPEAN PATENT SPECIFICATION

(11) **EP 2 750 102 B2**
(45) Date of publication and mention of the opposition decision: **10.12.2025**
(45) Mention of the grant of the patent: 15.03.2023
(21) Application number: 12462016.2
(22) Date of filing: 27.12.2012
(51) Int. Cl.: A61B 5/00

(54) **Method, system and computer readable medium for liver analysis**
Verfahren, System und computerlesbares Medium zur Leberanalyse
Procédé, système et support lisible par ordinateur pour l'analyse du foie

(43) Date of publication of application: 02.07.2014
(73) Proprietor: General Electric Company, Schenectady, NY 12345 (US)
(72) Inventor: Fidrich, Márta, 6722 Szeged (HU); Sprencz, Pál Csongor, 2040 Budaörs (HU); Bedekar, Devyani, Waukesha, WI (US); Fox, Amanda Jean, Waukesha, WI (US); Blaskovics, Tamás, 6722 Szeged (HU); Kovács, Ferenc, 6722 Szeged (HU); Kriston, András, 6722 Szeged (HU); Nyiri, Gergely, 6722 Szeged (HU); Bara, Norbert, 6722 Szeged (HU); Ruskó, Laszló, 6722 Szeged (HU)
(74) Representative: Fennell, Gareth Charles

(56) References cited:
- US-A1- 2007 055 455
- US-A1- 2008 103 385
- US-A1- 2010 111 396
- Handbook of the Medical Imaging Interaction Toolkit (MIT) software disclosed on the website :https://docsmitk org/2012.09/

## Description

### FIELD OF THE INVENTION

The subject matter disclosed herein relates to a method, a system and a computer readable medium for liver analysis. In a broader sense, the disclosed subject matter relates to image processing of medical images.

### BACKGROUND OF THE INVENTION

Segmentation and quantification of the liver, the liver segments and the tumors inside the liver are very important for oncologic diagnosis and therapy monitoring. In clinical practice, 3-dimensional medical images (CT, MR) are used for this purpose. In most cases the number, the location, the size, the shape and the density of tumors vary significantly, which makes oncologic liver assessment time consuming when each step is done manually. There is a need to facilitate this process making it available for the everyday clinical practice.

Detection of anatomical structures in medical images is a fundamental task in a number of clinical processes in the field of oncology, radiology and in planning surgical interventions. Exemplary techniques for imaging include conventional X-ray plane film radiography, computed tomography ("CT") imaging, magnetic resonance imaging ("MRI"), and nuclear medicine imaging techniques, such as positron emission tomography ("PET") and single photon emission computed tomography ("SPECT"). Detection is a prerequisite for measuring the size and shape of anatomical structures and to plan medical interventions. The spectrum of available detection/segmentation approaches is broad, ranging from manual outlining of structures in 2D cross-sections to more developed methods that use a so called 'registration' to find optimal correspondences between 3D images and a labeled probability map or anatomical atlas. There are also known semiautomatic approaches that combine the efficiency and repeatability of automatic segmentation with the human judgment that can only come from skilled expertise.

Quantification of liver diseases and tumors based on 3D medical images is an important problem of clinical diagnostics and therapy. Based on this information the best therapy can be selected and treatment efficiency can be monitored. Furthermore, the analysis of liver segments and tumors is very important for diagnostics as well as surgery planning.

Indicative measurements, like the whole liver volume; the position and size of liver segments; the total tumor burden of the liver; the location of tumors with respect to segments/lobes or vessels; the vascular anatomy of the liver; lesion or lobe specific spectral or perfusion analysis; are all important to enable, however, they are very challenging to realize using standard image processing tools.

The analysis of liver tumors is important for various cancer types because most of them (pulmonary, breast, colorectal) can develop liver metastases. Frequently, metastatic disease presents as a distribution of small lesions (2-10 mm) throughout the anatomy of the body. Tumor quantification requires precise tumor contours. The segmentation of all liver tumors is very challenging because the liver can involve multiple tumors (metastases as well as primers), which vary significantly in size, shape, density distribution. In many cases the liver contains so many tumors that makes it impossible (or very time consuming) to contour those manually. Even in an average clinical case, the liver contains tumors having irregular shape or ambiguous boundary, which introduces significant inter- and intra-operator differences in tumor quantification. In addition to the quantification of each tumor, the total tumor burden (ratio of the tumor volume and the total liver volume) of the liver is very important information for physician. The latter can be computed precisely if all tumors are precisely contoured; experienced physicians may estimate it based on visual assessment. Thus, there is also a need for a clinical application that automatically detects, segments and quantifies all (or as much as possible) tumors in the liver for diagnosis purposes.

As a general prior art, the following documents are referred to.

US 7046833 B2 and US 2002/0181754 A1 disclose region extracting methods for medical images.

US 2009/0097726 A1 disclose systems, methods and apparatus for automatic segmentation of liver in multiphase contrast-enhanced medical images, wherein multiphase liver segmentation methods are used based on a joint histogram of the phases.

US 7519209 B2 and US 2007/0055455 A1 disclose systems and methods of organ segmentation, based on a semi automatic iterative liver segmentation process.

US 8175354 B2 discloses a system and a method for interactive liver lobe segmentation. US 2008/0103385 A1 discloses a method and a system for liver lobe segmentation and pre-operative surgical planning.

US 2009/0003672 A1 discloses a method for segmenting image data for detecting a liver. This known liver segmentation method first segments the ribs and then assigns probability for each points inside the rib cage whether it belongs to the liver or not.

US 2009/0220137 A1 discloses an atlas based abdominal segmentation method, registering the atlas (the organs) to the patient.

US 2011/0052028 A1 discloses a method and a system of liver segmentation and segments separation.

WO 2005/055137 A2 and US 2010/0111396 A1 disclose vessel segmentation methods.

US 2006/0013482 A1 discloses a system and methods for organ segmentation and applications of same. This document describes an organ segmentation method based on geometric-deformable model framework. The segmentation starts from an initial contour, the speed function propagates the contour over homogenous regions to boundaries of organ.

US 2006/0052690A1 discloses a contrast agent imaging-driven health care system and method. This document describes a workflow where the inputs are contrasted data sets. A registering step is performed after the segmentation, and a fused image computed from the registered images can be used for classification or visualization.

US 2007/0116334 A1 discloses a method and an apparatus for three-dimensional interactive tools for semi-automatic segmentation. This document describes a method for segmenting anatomical objects from medical images. The method based on a shape-based interpolation.

US 2009/0052756 A1 discloses a system and a method for global-to-local shape matching for automatic liver segmentation in medical images. This document describes a method for automatically segmenting liver in digital medical images. The method is trained on a set of manually segmented liver. One seed point is needed to start the segmentation.

US 2007/0297561 A1 discloses a multi-component vessel segmentation. This document describes a multi component vessel segmentation method for CTA. In a first step the method segments the lumens of the blood vessels by thresholding. A detected lumen can be replaced by the mean value of the surrounding tissue and the complete organs can be segmented with advanced techniques such as 3D active objects.

US 2008/0247622 A1 discloses methods, systems and computer program products for hierarchical registration between a blood vessel and a tissue surface image for an subject. The method registers the generated vessel model and the tissue surface model by a hierarchical registration.

US 2009/0257630 A1 discloses a system and a method for interactive liver lobe segmentation. This document describes a method for separating a 3D liver object. One or more 3D separating surface is needed in the 3D space based on anatomical landmarks that are segmented automatically or interactively.

A common problem of the prior art solutions is that those do not allow-to carry out iterative decision steps, where one can step back or completely skip some steps. Furthermore, no optional advanced image analysis steps - such as liver fat quantification, perfusion computation or liver tumor segmentation step - are available in the known methods. Another problem with prior art methods is that many of them do not involve all functions needed for liver analysis.

These known systems and methods do not solve the problem of optimally combining automatic, mandatory user-interactive and optional steps for liver analysis.

Thus, there is a need for a solution allowing an improvement over existing methods and systems. There is a need for a liver analysis method, computer program and system eliminating as much as possible the shortcomings of known techniques. There is a particular need for a liver analysis method allowing an optimal combination of automated, interactive and optional steps, and final validation of the results. There is also a need for a method that eliminates the concerns about insufficient reliability of automated detection.

### SUMMARY OF THE INVENTION

According to the invention, a computer-implemented method for analyzing a liver is provided as defined in independent claim 1.

Further, according to the invention, a computer readable medium comprising a computer readable program is provided for liver analysis, wherein the computer readable program when executed on a computer causes the computer to perform the steps of the above method.

Further, according to the invention, a system for analyzing a liver, as defined in claim 10 is provided.

A liver analysis method according to the subject matter disclosed herein can decrease the total processing time and enables to establish an efficient clinical workflow, wherein certain steps of the workflow can be executed in a parallel manner. The subject matter disclosed herein allows a re-execution of detecting/defining/displaying steps based on a visual assessment in a very efficient way.

Liver quantification can be segment or lobe specific. The liver segment separation may be based on liver vessel (e.g. portal vein, hepatic artery, hepatic vein, biliary tree) to identify different segments. The method may use a seed point in a region growing method (e.g. based on Hessian features of the image) to segment the vessels and furthermore to separate the hepatic and portal vein structures automatically. Another way to separate liver segments or lobes is to use smooth surface(s) to partition the liver. The user defines the cutting surface by drawing curves on 2D slices of the corresponding medical image and the algorithm creates a 3D cutting surface by interpolation from the 2D cutting curves.

### BRIEF DESCRIPTION OF THE DRAWINGS

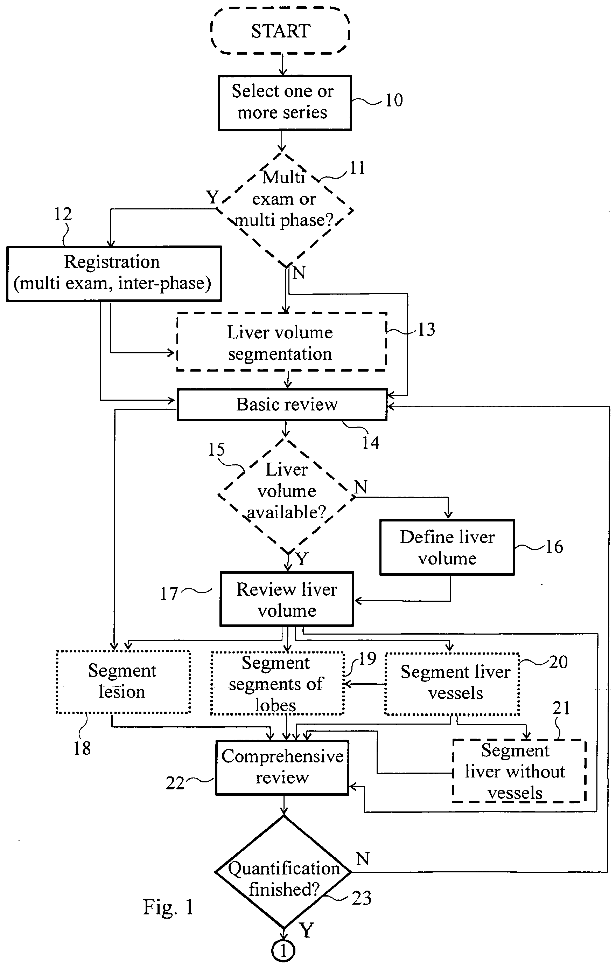

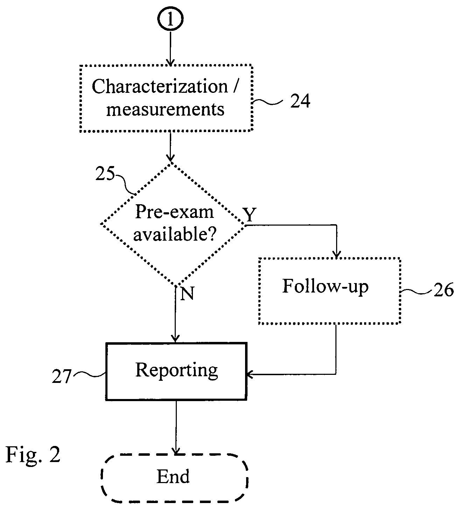

Characteristics, objectives and advantages of embodiments of the subject matter will become apparent from the following description, which is given solely by way of illustration and is non-limiting, and is to be read with reference to the appended drawing in which Figs. 1 and 2 illustrate a flowchart of a clinical workflow comprising automated, interactive and optional steps.

### DETAILED DESCRIPTION OF THE INVENTION

An embodiment may be embodied in the form of computer-implemented processes and apparatuses for practicing those processes. An embodiment may also be embodied in the form of a computer program code containing instructions embodied in tangible media, such as floppy diskettes, CD-ROMs, DVD-ROMs, hard drives, or any other computer readable storage medium, wherein, when the computer program code is loaded into and executed by a computer, the computer becomes an apparatus for carrying out the method. An embodiment may also be embodied in the form of computer program code, for example, whether stored in a storage medium, loaded into and/or executed by a computer, or transmitted over some transmission medium, such as over electrical wiring or cabling, through fiber optics, or via electromagnetic radiation, wherein when the computer program code is loaded into and executed by a computer, the computer becomes an apparatus for carrying out the method. When implemented on a general-purpose microprocessor, the computer program code segments configure the microprocessor to create specific logic circuits.

Furthermore, an embodiment can take the form of a computer program product accessible from a computer-usable or computer-readable medium providing program code for use by or in connection with a computer or any instruction execution system. For the purposes of this description, a computer-usable or computer readable medium - can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium.

The subject matter disclosed herein describes a workflow of analyzing liver. The disclosure proposes a framework that facilitates liver analysis, comprising e.g. detection, segmentation, and quantification steps of all tumors within the liver or in its lobes or segments. A typical user is a clinical radiologist or oncologist, who wants to objectively characterize the liver. The method is divided into separate branches, which allow building an optimal framework from the user's point of view.

The objective quantification of the above measurements can significantly increase the reliability of the diagnosis or the effectiveness of the therapy. The manual segmentation of liver segments, vessels and lesions is very challenging because the liver can involve multiple tumors, it can be affected by various diseases (cirrhosis), the anatomy of the liver and the level of contrast-enhancement can be very different among patients, which can lead to significant inter-operator variability.

In order to facilitate such a complex analysis, the subject matter disclosed herein describes a workflow, which integrates numerous features to perform segmentation tasks more efficiently, automates several measurements, provides complex measurements incorporating more findings, and allows advanced analysis for all findings. This way, the disclosed system is a complete package that can speed up a wide range of activities related to clinical liver assessment.

An embodiment of the workflow contains the following steps as depicted in Figs. 1 and 2. Rectangles with dashed line frames represent automated functions, rectangles with continuous line frames represent mandatory interactive user actions, while rectangles with dotted line frames represent optional steps.

Figs. 1 and 2 describe a workflow covering all possible steps that can be applied to an exam. The input is one or more multi-phase CT or MR exam. The complete workflow allows executing all features on a contrast-enhanced image, furthermore, it allows skipping steps according to user needs. The result of each step can be visualized and stored. It is also possible to process non-contrast image series. In such cases contrast specific features (vessel segmentation, vessel-based segment separation) are not available. Any segmented structure or measurement generated during the workflow can be visualized and stored.

As a first mandatory, user interactive step 10, the user selects a set of medical images to be processed. It is possible to load a single image (i.e. the set may consist of one image only) or multiple images, exams or series (in the following: set of medical images).

Next, an automated selection between two options is carried out in step 11. The selected exams are processed differently depending on the number and type of exams. If multi-phase or multi-date exam is loaded, the system proceeds with step 12, otherwise it proceeds with step 13 and in parallel the user proceeds with step 14.

Step 12 is mandatory if a multi-phase or multi-date exam is loaded. The input set of medical images are registered in this step using an automated, a semi automated or a manual method. Then, the system proceeds with step 13 and in parallel the user proceeds with step 14.

Step 13 is an automated segmentation method defining the volume of the liver. The method runs in background.

Next, the user carries out a basic review on the set of medical images and the already available segmented structures in various (2D/3D) views in step 14, which is a mandatory user interactive step. The user visually evaluates the exams and takes measurements, e.g. relating to volume, diameter and/or density. The user can proceed with step 15 or step 18.

In automated step 15, the system checks if the liver volume is available, i.e. an appropriate liver volume is obtained by the automated segmentation step 13. If yes, the system proceeds with step 17. If not, the user manually defines liver volume using interactive tools in step 16, which is a mandatory, user interactive step. Next, the system proceeds with step 17.

Step 17 is a mandatory, user interactive step, in which the liver volume is displayed in 2D and/or 3D views. The user can review and manually edit the liver volume, if needed. From this step the user can proceed with step 18, step 19 or step 20 carrying out user selected further segmentations, or go directly to the review step 22. It is an advantage of the method and system that the user selected further segmentations are carried out in the light of the already segmented liver volume, so no lesions and structures outside the liver are taken into account for further proceedings. So, prior to carrying out the user selected further segmentations, the liver volume reviewing step 17 is carried out enabling the user to review and manually edit the liver volume. When the control subsequently arrives to this workflow branch again, the system can automatically skips steps 15, 16, and 17, if step 17 was already executed by the user.

If step 18 is chosen, the user contours liver lesions using automated or semi automated methods, and proceeds with step 22.

If step 19 is chosen, the user can separate the liver into segments or lobes. If step 20 is already executed, a vessel-based approach is available. In such case the segmental branches of the portal vein are automatically or manually labeled and the system computes the segments or lobes. So, the results of the liver vessel segmentation step 20 may be used to carry out the liver lobe segmentation step 19. Otherwise, the user defines cutting planes (surfaces) to separate segments/lobes, and the system computes segments based on these planes. The segments are displayed in 2D/3D views, and the user can merge segments into one volume. If done, the user proceeds with step 22.

If step 20 is chosen, liver vessel structures, e.g. portal/hepatic vein, hepatic artery, are segmented automatically or semi automatically. The user can optionally proceed with step 19, step 21, or step 22.

In automated step 21, the liver volume without vessel structures is automatically computed as a separate segmented volume. This step is particularly advantageous as a considerable part of the volume enclosing the liver is filled by vessels (e.g. veins and arteries), and in this way the volume occupied by the liver tissue can be obtained.

It is an advantage of the subject matter disclosed herein that it enables the segmentation of the lesions with respect of separate liver lobes. Thus, a more concentrated action can be planned by the surgeon.

In a mandatory, user interactive comprehensive review step 22 the user reviews all available findings. These findings may involve lesions, whole liver, liver segments, vessel structures, etc. The review may comprise visual assessment and evaluation of the existing measurements in the form of a table or any suitable information summary.

If - after the comprehensive review step 22 - the user wishes to perform further processing of the set of medical images (e.g. segmentation and/or quantification of additional findings), a selective returning step 23 enables to return to step 14. If not, the system proceeds with step 24. 1. So, after the comprehensive reviewing step 22, a selective returning to the basic review step 14 is possible if the comprehensive review indicates that further processing of the set of medical images is necessary.

In optional step 24, the user may generate various complex measurements incorporating different findings (e.g. liver tumor burden, liver volume without vessels, etc), or perform advanced analysis for some findings (e.g. spectral analysis or fat quantification in case of spectral CT exams, perfusion measurements, etc).

In a further optional selection step 25, if no processed exam belonging to a prior date is available, the user proceeds with step 27. Otherwise, in optional step 26, the current exam is automatically registered with the previously processed data, the corresponding findings are identified, and the difference of measurements is computed and displayed.

In a mandatory reporting step 27, the system generates a report and saves all findings, measurements and/or other results. Thus, if no returning to the basic review step 14 is carried out, the report is generated for the findings, measurements and/or other results.

An advantage of the subject matter disclosed herein is that all the main functions (liver volume and vessel segmentation, liver segment separation, virtual scalpel, tumor segmentation) of an optimal workflow are implemented and evaluated. The system and method can also be applied to any 3-dimensional modality images, and can facilitate a wide range of image based liver analysis, so it can be applied on various clinical areas, e.g. radiology, oncology or surgery.

A further advantage of the subject matter disclosed herein is that it allows to carry out user defined workflows, i.e. it is possible to omit steps which are not needed in certain clinical scenarios. The disclosed system and method involve all important segmentation/quantification steps needed for liver analysis; these steps are integrated into a complete clinical review process, and may be extended with advanced analysis like spectral CT tissue characterization or follow-up. Furthermore, the disclosed system and method run liver volume segmentation in the background, while the user performs the basic review. This enables a high efficiency and flexibility.

A still further advantage of the subject matter disclosed herein is that it involves all important functions of liver analysis in an optimal order, which allows the user to perform a very complex process in an easy way.

The user of the system or the method can be a radiologist, an oncologist, or a surgeon. The basis of the complete workflow is the fully automated liver volume segmentation. The other optional features are liver lesion contouring, portal vein segmentation, vessel- and plane- (i.e. smooth surface) based segment/lobe separation. The proposed workflow allows the user to customize the workflow (involve or omit certain steps according to the particular needs), which makes the system and the method efficient in clinical routine.

An exemplary embodiment is a computer readable medium comprising a computer readable program for liver analysis, wherein the computer readable program when executed on a computer causes the computer to perform the steps of the above disclosed method.

Another exemplary embodiment is a system for liver analysis, the system comprising units configured to perform the steps of the above disclosed method. These units can take the form of an entirely hardware embodiment, an entirely software embodiment or an embodiment including both hardware and software elements.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art.

## Claims

1. A computer-implemented method for analyzing a liver, the method comprising the steps of:
- enabling a user to select (10) a set of medical images to be processed;
- automatically segmenting (13) the liver in the set of medical images to define the volume of the liver;
- enabling the user to carry out a basic review (14) on the set of medical images and the result of the segmentation, the basic review comprising visual evaluation and the taking of measurements relating to at least one of volume, diameter and density;
- a liver volume reviewing step (17), wherein a user is able to review and manually edit the liver volume;
- after the liver volume reviewing step (17), enabling the user to carry out user selected further segmentations comprising at least one of lesion segmentations (18), liver lobe segmentations (19) and liver vessel segmentation (20);
- enabling the user in a comprehensive review step (22) to review all available findings, and subsequently, enabling the user to selectively return (23) to the basic review step (14) if the comprehensive review indicates that further processing of the set of medical images is necessary; and
- after returning to the basic review step (14), automatically skipping the liver volume reviewing step (17) if the liver volume reviewing step (17) was already executed by the user.

2. The method according to claim 1, **characterized in that** the comprehensive review step (22) comprises visual assessment and evaluation of the existing measurements in the form of a table or an information summary.

3. The method according to claim 1**,** wherein one of the user selected further segmentations comprises a liver vessel segmentation (20), the method **characterized in** enabling the results of said liver vessel segmentation (20) to be used to carry out said liver lobe segmentation (19).

4. The method according to any of claims 1 to 3, **characterized by** automatically checking (15) - after the basic review step (14) - whether an appropriate liver volume is obtained by the automated segmentation step (13) and enabling manual definition of the liver volume if an appropriate liver volume is not obtained.

5. The method according to any of claims 1 to 4, **characterized by** generating measurements or performing analysis - after the selectively returning (23) step - wherein said measurements relate to liver volume without vessels or perfusion, and performing analysis comprises performing analysis for findings, preferably a spectral analysis or a fat quantification in case of spectral CT exams.

6. The method according to any of claims 1 to 5, **characterized in that** if a processed set of medical images belonging to a prior date is available, the current set of medical images is registered with the previously processed data, and preferably the corresponding findings are identified, and the difference of measurements is computed and displayed.

7. The method according to any of claims 1 to 6, **characterized in that** if no returning to the basic review step (14) is carried out, a report is generated (27) for the findings, measurements and/or other results.

8. The method according to any of claims 1 to 7, **characterized by** registering (12) the set of medical images in case if a multi-phase or multi-date set of medical images is selected in said selecting step (10).

9. A computer readable medium comprising a computer readable program for diagnosing a liver, wherein the computer readable program when executed on a computer causes the computer to perform the steps of the method according to any of claims 1 to 8.

10. A system for analyzing a liver, the system comprising
- a selecting unit configured to enable the user to select (10) a set of medical images to be processed;
- an automatic segmenting unit configured to automatically segment (13) the liver in the set of medical images to define the volume of the liver;
- a basic review unit configured to enable a user to carry out a basic review (14) on the set of medical images and the result of the segmentation, the basic review comprising visual evaluation and the taking of measurements relating to at least one of volume, diameter and density;
- a liver volume reviewing unit configured to enable a review step (17), wherein a user is able to review and manually edit the liver volume;
- segmentation units configured, after the liver volume reviewing step (17), to carry out user selected further segmentations, the segmentation units comprising at least one of a lesion segmentation (18) unit, a liver lobe segmentation (19) unit and a liver vessel segmentation (20) unit;
- a comprehensive review unit configured to enable the user in a comprehensive review step (22) to review all available findings; and
- a selective returning unit configured to allow the user to selectively return (23) to the basic reviewing step (14) after the comprehensive review step (22), if the comprehensive review (22) indicates that further processing of the set of medical images is necessary;
- wherein the system is configured to automatically skip the liver volume reviewing step (17) after returning to the basic reviewing step (14) if the liver volume reviewing step (17) was already executed by the user.

11. The system according to claim 10, **characterized in that** the comprehensive review unit is configured to enable the user to perform visual assessment and evaluation of the measurements in the form of a table or an information summary.

12. The system according to claim 11, wherein one of the segmentation units comprises a liver vessel segmentation (20) unit, the system **characterized in that** the output of said liver vessel segmentation (20) is supplied as an input for said liver lobe segmentation (19) unit.

13. The system according to any of claims 10 to 12, **characterized by** comprising an automatic checking unit configured to automatically check (15) - after the basic review (14) - whether an appropriate liver volume is obtained by the automated segmentation (13) and enabling manual definition of the liver volume if an appropriate liver volume is not obtained.

14. The system according to any of claims 10 to 13, **characterized by** comprising a unit configured to generate measurements or perform analysis - after the selective return (23) to the basic review (14) - wherein said measurements relate to liver tumor burden, liver volume without vessels or perfusion, and performing analysis comprises performing analysis for findings, preferably a spectral analysis or a fat quantification in case of spectral CT exams.

15. The system according to any of claims 10 to 14, **characterized by** comprising a unit configured to register the set of medical images with previously processed data, and preferably configured to identify corresponding findings, and to compute and display the difference of measurements.

16. The system according to any of claims 10 to 15, **characterized by** comprising a report generating unit configured to generate (27) a report for the findings, measurements and/or other results.

17. The system according to any of claims 10 to 16, **characterized by** comprising a registering unit configured to register (12) the set of medical images if a multiphase or multi-date set of medical images is selected in said selecting step (10).

## Patentansprüche

1. Computerimplementiertes Verfahren zur Analyse einer Leber, wobei das Verfahren die Schritte umfasst:
- Ermöglichen, dass ein Benutzer einen Satz zu verarbeitender medizinischer Bilder auswählt (10);
- automatisches Segmentieren (13) der Leber in dem Satz medizinischer Bilder, um das Volumen der Leber zu definieren;
- Ermöglichen, dass der Benutzer eine grundlegende Prüfung (14) an dem Satz der medizinischen Bilder und dem Ergebnis der Segmentierung durchführt, wobei die grundlegende Prüfung visuelle Evaluierung und das Durchführen von Messungen in Bezug auf mindestens eines von Volumen, Durchmesser und Dichte umfasst;
- einen Lebervolumenprüfschritt (17), wobei ein Benutzer in der Lage ist, das Lebervolumen zu prüfen und manuell zu editieren;
- nach dem Lebervolumenprüfschritt (17) Ermöglichen, dass der Benutzer benutzerausgewählte weitere Segmentierungen durchführt, umfassend mindestens eine von Läsionssegmentierungen (18), Leberlappensegmentierungen (19) und Lebergefäßsegmentierung (20);
- Ermöglichen, dass der Benutzer in einem umfassenden Prüfschritt (22) alle verfügbaren Befunde prüft, und nachfolgendes Ermöglichen, dass der Benutzer selektiv zu dem grundlegenden Prüfschritt (14) zurückkehrt (23), falls der umfassende Prüfschritt angibt, dass weitere Verarbeitung des Satzes medizinischer Bilder erforderlich ist; und
- nach der Rückkehr zu dem grundlegenden Prüfschritt (14) automatisches Überspringen des Lebervolumenprüfschritts (17), falls der Lebervolumenprüfschritt (17) von dem Benutzer bereits ausgeführt wurde.

2. Verfahren nach Anspruch 1, **dadurch gekennzeichnet, dass** der umfassende Prüfschritt (22) visuelle Beurteilung und Evaluierung vorhandener Messungen in Form einer Tabelle oder einer Informationszusammenfassung umfasst.

3. Verfahren nach Anspruch 1, wobei eine der benutzerausgewählten weiteren Segmentierungen eine Lebergefäßsegmentierung (20) umfasst, wobei das Verfahren **dadurch gekennzeichnet ist, dass** ermöglicht wird, dass die Ergebnisse der Lebergefäßsegmentierung (20) zu verwenden sind, um die Leberlappensegmentierung (19) durchzuführen.

4. Verfahren nach einem der Ansprüche 1 bis 3, **dadurch gekennzeichnet, dass** - nach dem grundlegenden Prüfschritt (14) - automatisch kontrolliert (15) wird, ob durch den automatischen Segmentierungsschritt (13) ein geeignetes Lebervolumen erhalten wird, und dass manuelle Definition des Lebervolumens ermöglicht wird, falls kein geeignetes Lebervolumen erhalten wird.

5. Verfahren nach einem der Ansprüche 1 bis 4, **dadurch gekennzeichnet, dass** - nach dem selektiven Rückkehrschritt (23) - Messungen erstellt oder Analyse durchgeführt wird, wobei die Messungen das Lebervolumen ohne Gefäße oder Perfusion betreffen, und dass Durchführen von Analyse Durchführen von Analyse auf Befunde, vorzugsweise im Fall von spektralen CT-Untersuchungen eine Spektralanalyse oder eine Fettquantifizierung umfasst.

6. Verfahren nach einem der Ansprüche 1 bis 5, **dadurch gekennzeichnet, dass**, falls ein verarbeiteter Satz medizinischer Bilder verfügbar ist, der zu einem früheren Datum gehört, der aktuelle Satz medizinischer Bilder mit den zuvor verarbeiteten Daten registriert wird und vorzugsweise die entsprechenden Befunde identifiziert werden und die Differenz der Messungen berechnet und angezeigt wird.

7. Verfahren nach einem der Ansprüche 1 bis 6, **dadurch gekennzeichnet, dass**, falls keine Rückkehr zu dem grundlegenden Prüfschritt (14) durchgeführt wird, ein Bericht (27) über die Befunde, Messungen und/oder sonstigen Ergebnisse erstellt wird.

8. Verfahren nach einem der Ansprüche 1 bis 7, **gekennzeichnet durch** Registrieren (12) des Satzes medizinischer Bilder, falls ein mehrphasiger oder mehrere Datumsangaben betreffender Satz medizinischer Bilder in dem Auswahlschritt (10) ausgewählt wird.

9. Computerlesbares Medium, umfassend ein computerlesbares Programm zum Diagnostizieren einer Leber, wobei das computerlesbare Programm, wenn es auf einem Computer ausgeführt wird, bewirkt, dass der Computer die Schritte des Verfahrens nach einem der Schritte 1 bis 8 durchführt.

10. System zum Analysieren einer Leber, wobei das System umfasst:
- eine Auswahleinheit, die ausgestaltet ist, um zu ermöglichen, dass der Benutzer einen Satz zu verarbeitender medizinischer Bilder auswählt (10);
- eine automatische Segmentierungseinheit, die ausgestaltet ist zum automatischen Segmentieren (13) der Leber in dem Satz medizinischer Bilder, um das Volumen der Leber zu definieren;
- eine grundlegende Prüfeinheit, die ausgestaltet ist, um zu ermöglichen, dass ein Benutzer eine grundlegende Prüfung (14) an dem Satz der medizinischen Bilder und dem Ergebnis der Segmentierung durchführt, wobei die grundlegende Prüfung visuelle Evaluierung und das Durchführen von Messungen in Bezug auf mindestens eines von Volumen, Durchmesser und Dichte umfasst;
- eine Lebervolumenprüfeinheit, die ausgestaltet ist, um einen Prüfschritt (17) zu ermöglichen, wobei ein Benutzer in der Lage ist, das Lebervolumen zu prüfen und manuell zu editieren;
- Segmentierungseinheiten, die ausgestaltet sind, um nach dem Lebervolumenprüfschritt (17) benutzerausgewählte weitere Segmentierungen durchzuführen, wobei die Segmentierungseinheiten mindestens eine von einer Einheit zur Läsionssegmentierung (18), einer Einheit zur Leberlappensegmentierung (19) und einer Einheit zur Lebergefäßsegmentierung (20) umfassen;
- eine umfassende Prüfeinheit, die ausgestaltet ist, um zu ermöglichen, dass der Benutzer in einem umfassenden Prüfschritt (22) alle verfügbaren Befunde prüft; und
- eine selektive Rückkehreinheit, die ausgestaltet ist, um dem Benutzer das selektive Rückkehren (23) zu dem grundlegenden Prüfschritt (14) nach dem umfassenden Prüfschritt (22) zu gestatten, falls die umfassende Prüfung (22) angibt, dass weitere Verarbeitung des Satzes medizinischer Bilder erforderlich ist;
- wobei das System ausgestaltet ist, um den Lebervolumenüberprüfungsschritt (17) nach der Rückkehr zu dem grundlegenden Prüfschritt (14) automatisch zu überspringen, falls der Lebervolumenprüfschritt (17) von dem Benutzer bereits ausgeführt wurde.

11. System nach Anspruch 10, **dadurch gekennzeichnet, dass** die umfassende Prüfeinheit ausgestaltet ist, um dem Benutzer das Durchführen von visueller Beurteilung und Evaluierung der Messungen in Form einer Tabelle oder einer Informationszusammenfassung zu ermöglichen.

12. System nach Anspruch 11, wobei eine der Segmentierungseinheiten eine Einheit zur Lebergefäßsegmentierung (20) umfasst, wobei das System **dadurch gekennzeichnet ist, dass** die Ausgabe der Lebergefäßsegmentierung (20) als Eingabe für die Einheit zur Leberlappensegmentierung (19) verwendet wird.

13. System nach einem der Ansprüche 10 bis 12, **dadurch gekennzeichnet, dass** es eine automatische Kontrolleinheit umfasst, die ausgestaltet ist, um - nach dem grundlegenden Prüfschritt (14) - automatisch zu kontrollieren (15), ob durch die automatische Segmentierung (13) ein geeignetes Lebervolumen erhalten wird, und dass manuelle Definition des Lebervolumens ermöglicht wird, falls kein geeignetes Lebervolumen erhalten wird.

14. System nach einem der Ansprüche 10 bis 13, **dadurch gekennzeichnet, dass** es eine Einheit umfasst, die ausgestaltet ist, um - nach dem selektiven Rückkehrschritt (23) zu der grundlegenden Prüfung (14) - Messungen zu erstellen oder Analyse durchzuführen, wobei die Messungen die Lebertumorlast, das Lebervolumen ohne Gefäße oder Perfusion betreffen, und dass Durchführen von Analyse Durchführen von Analyse auf Befunde, vorzugsweise im Fall von spektralen CT-Untersuchungen eine Spektralanalyse oder eine Fettquantifizierung umfasst.

15. System nach einem der Ansprüche 10 bis 14, **dadurch gekennzeichnet, dass** es eine Einheit umfasst, die ausgestaltet ist, um den Satz medizinischer Bilder mit zuvor verarbeiteten Daten zu registrieren, und vorzugsweise ausgestaltet ist, um entsprechende Befunde zu identifizieren und um die Differenz der Messungen zu berechnen und anzuzeigen.

16. System nach einem der Ansprüche 10 bis 15, **dadurch gekennzeichnet, dass** es eine Berichterstellungseinheit umfasst, die ausgestaltet ist, um einen Bericht über die Befunde, Messungen und/oder sonstigen Ergebnisse zu erstellen (27).

17. System nach einem der Ansprüche 10 bis 16, **dadurch gekennzeichnet, dass** es eine Registriereinheit umfasst, die ausgestaltet ist, um den Satz medizinischer Bilder zu registrieren (12), falls ein mehrphasiger oder mehrere Datumsangaben betreffender Satz medizinischer Bilder in dem Auswahlschritt (10) ausgewählt wird.

## Revendications

1. Procédé mis en œuvre par ordinateur pour analyser un foie, le procédé comprenant les étapes suivantes :
- permettre à un utilisateur de sélectionner (10) un ensemble d'images médicales à traiter ;
- segmenter automatiquement (13) le foie dans l'ensemble d'images médicales pour définir le volume du foie ;
- permettre à l'utilisateur d'effectuer un examen de base (14) sur l'ensemble d'images médicales et le résultat de la segmentation, l'examen de base comprenant une évaluation visuelle et la prise de mesures relatives à au moins un élément parmi le volume, le diamètre et la densité ;
- une étape d'examen du volume de foie (17), un utilisateur étant capable d'examiner et de modifier manuellement le volume de foie ;
- après l'étape d'examen du volume de foie (17), permettre à l'utilisateur d'effectuer des segmentations supplémentaires sélectionnées par l'utilisateur comprenant au moins une des segmentations de lésions (18), des segmentations de lobes du foie (19) et des segmentations de vaisseaux du foie (20) ;
- permettre à l'utilisateur, dans une étape d'examen complet (22), d'examiner toutes les observations disponibles, et, par la suite, de revenir sélectivement (23) à l'étape d'examen de base (14) si l'examen complet indique qu'un traitement supplémentaire de l'ensemble d'images médicales est nécessaire ;
- après le retour à l'étape d'examen de base (14), sauter automatiquement l'étape d'examen du volume de foie (17) si l'étape d'examen du volume de foie (17) a déjà été exécutée par l'utilisateur.

2. Procédé selon la revendication 1, **caractérisé en ce que** l'étape d'examen complet (22) comprend une évaluation visuelle et une évaluation des mesures existantes sous la forme d'un tableau ou d'un résumé d'informations.

3. Procédé selon la revendication 1, l'une des segmentations supplémentaires sélectionnées par l'utilisateur comprenant une segmentation des vaisseaux du foie (20), le procédé étant **caractérisé en ce qu'**il permet aux résultats de ladite segmentation des vaisseaux du foie (20) d'être utilisés pour effectuer ladite segmentation du lobe du foie (19).

4. Procédé selon l'une quelconque des revendications 1 à 3, **caractérisé par** la vérification automatique (15) - après l'étape d'examen de base (14) - si un volume de foie approprié est obtenu par l'étape de segmentation automatisée (13) et permettant la définition manuelle du volume de foie si un volume de foie approprié n'est pas obtenu.

5. Procédé selon l'une quelconque des revendications 1 à 4, **caractérisé par** la génération de mesures ou la réalisation d'un examen - après l'étape de renvoi sélectif (23) - lesdites mesures se rapportant au volume de foie sans vaisseaux ni perfusion, et la réalisation d'un examen comprenant la réalisation d'un examen des observations, de préférence un examen spectral ou une quantification de la graisse dans le cas d'examens CT spectraux.

6. Procédé selon l'une quelconque des revendications 1 à 5, **caractérisé en ce que** si un ensemble d'images médicales traité appartenant à une date antérieure est disponible, l'ensemble d'images médicales actuel est enregistré avec les données traitées précédemment, et de préférence les observations correspondantes sont identifiées, et la différence des mesures est calculée et affichée.

7. Procédé selon l'une quelconque des revendications 1 à 6, **caractérisé en ce que** si aucun retour à l'étape d'examen de base (14) n'est effectué, un rapport est généré (27) pour les observations, les mesures et/ou les autres résultats.

8. Procédé selon l'une quelconque des revendications 1 à 7, **caractérisé par** l'enregistrement (12) de l'ensemble d'images médicales dans le cas où un ensemble d'images médicales multi-phases ou multi-dates est sélectionné dans ladite étape de sélection (10).

9. Support lisible par ordinateur comprenant un programme lisible par ordinateur pour diagnostiquer un foie, le programme lisible par ordinateur, lorsqu'il est exécuté sur un ordinateur, amenant l'ordinateur à réaliser les étapes du procédé selon l'une quelconque des revendications 1 à 8.

10. Système d'analyse d'un foie, le système comprenant :
- une unité de sélection configurée pour permettre à l'utilisateur de sélectionner (10) un ensemble d'images médicales à traiter ;
- une unité de segmentation automatique configurée pour segmenter automatiquement (13) le foie dans l'ensemble d'images médicales pour définir le volume du foie ;
- une unité d'examen de base configurée pour permettre à un utilisateur d'effectuer un examen de base (14) sur l'ensemble d'images médicales et le résultat de la segmentation, l'examen de base comprenant une évaluation visuelle et la prise de mesures relatives à au moins un élément parmi le volume, le diamètre et la densité ;
- une unité d'examen du volume de foie configurée pour permettre une étape d'examen (17), un utilisateur étant capable d'examiner et de modifier manuellement le volume de foie ;
- des unités de segmentation configurées, après l'étape d'examen du volume de foie (17), pour effectuer des segmentations supplémentaires sélectionnées par l'utilisateur, les unités de segmentation comprenant au moins une unité de segmentation des lésions (18), une unité de segmentation des lobes du foie (19) et une unité de segmentation des vaisseaux du foie (20) ;
- une unité d'examen complet configurée pour permettre à l'utilisateur dans une étape d'examen complet (22) d'examiner toutes les observations disponibles ; et
- une unité de retour sélectif configurée pour permettre à l'utilisateur de revenir sélectivement (23) à l'étape d'examen de base (14) après l'étape d'examen complet (22), si la revue complète (22) indique qu'un traitement supplémentaire de l'ensemble d'images médicales est nécessaire ;
- le système étant configuré pour sauter automatiquement l'étape d'examen du volume de foie (17) après le retour à l'étape d'examen de base (14) si l'étape d'examen du volume de foie (17) a déjà été exécutée par l'utilisateur.

11. Système selon la revendication 10, **caractérisé en ce que** l'unité d'examen complet est configurée pour permettre à l'utilisateur d'effectuer une évaluation visuelle et une évaluation des mesures sous la forme d'un tableau ou d'un résumé d'informations.

12. Système selon la revendication 11, l'une des unités de segmentation comprenant une unité de segmentation des vaisseaux du foie (20), le système étant **caractérisé en ce que** la sortie de ladite segmentation des vaisseaux du foie (20) est fournie en tant qu'entrée pour ladite unité de segmentation du lobe du foie (19).

13. Système selon l'une quelconque des revendications 10 à 12, **caractérisé en ce qu'**il comprend une unité de vérification automatique configurée pour vérifier automatiquement (15) - après l'examen de base (14) - si un volume de foie approprié est obtenu par la segmentation automatisée (13) et permettant la définition manuelle du volume de foie si un volume de foie approprié n'est pas obtenu.

14. Système selon l'une quelconque des revendications 10 à 13, **caractérisé en ce qu'**il comprend une unité configurée pour générer des mesures ou effectuer un examen - après le retour sélectif (23) à l'examen de base (14) - lesdites mesures se rapportant à la charge tumorale du foie, au volume de foie sans vaisseaux ou à la perfusion, et la réalisation de l'examen comprenant la réalisation d'un examen des observations, de préférence un examen spectral ou une quantification de la graisse dans le cas d'examens CT spectraux.

15. Système selon l'une quelconque des revendications 10 à 14, **caractérisé en ce qu'**il comprend une unité configurée pour enregistrer l'ensemble d'images médicales avec des données traitées précédemment, et de préférence configurée pour identifier les observations correspondantes, et pour calculer et afficher la différence de mesures.

16. Système selon l'une quelconque des revendications 10 à 15, **caractérisé en ce qu'**il comprend une unité de génération de rapport configurée pour générer (27) un rapport sur les observations, les mesures et/ou les autres résultats.

17. Système selon l'une quelconque des revendications 10 à 16, **caractérisé en ce qu'**il comprend une unité d'enregistrement configurée pour enregistrer (12) l'ensemble d'images médicales si un ensemble d'images médicales multi-phases ou multi-dates est sélectionné dans ladite étape de sélection (10).
